# EUROPEAN PATENT APPLICATION

(11) **EP 4 851 277 A1**
(43) Date of publication of application: **09.12.2026**
(21) Application number: 25748206.7
(22) Date of filing: 29.01.2025
(51) Int. Cl.: C12N 1/20, C05F 11/08, C05F 17/20, C12R 1/07

(54) **BACILLUS NAKAMURAI CECT 30581 AND PLANT GROWTH-PROMOTING METABOLIC PRODUCTS THEREOF UNDER CONDITIONS OF WATER STRESS**

(30) Priority: 30.01.2024 ES 202430067
(71) Applicant: Biobab R&D, S.L., 28670 Madrid (ES)
(72) Inventor: Gutierrez Albanchez, Enrique, 28668 Alcorcón, Madrid (ES); Ranceschini Sarria, André Lucio, 28821 Coslada, Madrid (ES); Horche Trueba, Ignacio, 28925 Alcorcón, Madrid (ES); García-Villaraco Velasco, Ana, 28007 Madrid (ES); Lucas Garcia, José Antonio, 28760 Tres Cantos, Madrid (ES); Ramos Solano, Beatriz, 28224 Pozuelo de Alarcón, Madrid (ES); Gutierrez Mañero, Francisco Javier, 28691 Villanueva de la Cañada, Madrid (ES)
(86) International application number: PCT/ES2025/070036
(87) International publication number: WO 2025/163226

(57) **Abstract**

Bacterial strain *Bacillus nakamurai* CECT 30581, a microorganism of the group of Gram + bacteria, genus *Bacillus,* and the metabolic products resulting from its fermentation in the culture medium, total metabolic fluid (TMF), hexanic fraction (HF) and hexanic subfraction (HS), stimulants of the absorption of nitrogen, phosphorus, potassium and manganese in plants, and of the fixation of atmospheric CO2 and transpiration, even under conditions of water stress. Once this strain was isolated from the rhizosphere of Pinus pinaster, and characterized from a physiological and genetic point of view, the TMF was separated/extracted and the metabolites responsible for its effects were identified, as a characterization profile of the bacterial products for their different uses. Both the bacteria and their compositions can serve as a basis for the preparation of various preparations intended to optimize the photosynthetic process of plants, improving plant growth and production in adverse situations of drought or reduction of irrigation water, and also, within the field of organic biofertilizers, to improve mineral nutrition in the indicated nutrients.

## Description

The present invention relates to a new strain of *Bacillus nakamurai* and the metabolites produced during its fermentation process, both components to be used in the treatment of plants subjected to water stress conditions, with the aim of improving mineral nutrition in nitrogen, phosphorus, potassium and manganese, and improving the uptake and fixation of CO2, increasing biomass production (dry weight), water use efficiency, and plant production in drought environments or lack of water at risk.

This strain has been deposited for patent purposes in the Spanish Collection of Type Cultures (CECT), dated March 29, 2022, where it has been assigned the deposit number 30581. The CECT is located in the research building of the University of Valencia, located on the Burjassot campus (DP 46100 - Valencia, Spain).

### TECHNICAL FIELD. -

This invention is situated in the field of agri-food biotechnology, specifically within the field of plant growth promoting bacteria (PGPB), since the aforementioned bacterial strain and its metabolites can serve as a basis for the preparation of various products intended to increase CO2 uptake and optimize the photosynthetic process of plants, which translates into an increase in dry weight and production.

And also in the field of organic biofertilizers, since these bacterial products can be used to improve the mineral nutrition of plants in these nutrients.

### STATE OF THE ART.-

Nitrogen (N) and carbon (C) are essential elements for plant development and crop production. Improving the utilization of these elements can increase agricultural productivity and reduce the need for fertilizers. While N is mainly supplied through the roots, CO2 is obtained through the process of photosynthesis. Photosynthesis is a vital process in plant physiology, where light energy is converted into chemical energy: H₂O + CO₂ + E (light energy) produces C₆H₁₂O₆ + O₂. This process involves the absorption of water through the roots and the uptake of carbon dioxide in the leaves, and the capture of energy from solar radiation. Photosynthesis is mainly composed of two stages: first, the capture of light energy and its conversion into chemical energy (in the form of ATP and NADPH), and second, the use of this energy to fix CO₂ and build organic compounds that will provide the energy for their vital processes.

During photosynthesis, photosynthetic pigments play an essential role in capturing light energy, but this process also generates oxidative stress in the form of free radicals, mainly reactive oxygen species (ROS). The plant has mechanisms to eliminate these free radicals, these processes are inducible and are essential to keep them within adequate physiological levels and to prevent them from causing structural damage at the cellular level that implies a loss of performance in all the vital processes of the plant.

A critical aspect in this entire process is the adequate availability of water and the correct hydration of the plant since the uptake of CO₂ necessarily implies a loss of water through the stomata (transpiration). This transpiration process tends to accelerate under adverse conditions of temperature and humidity. Therefore, there is a contradiction in the requirements of photosynthesis, since a good uptake of CO₂ through the stomata is essential, but at the same time, it can result in a significant loss of water under unfavorable conditions.

Thus, it is essential that the plant is adequately hydrated. The key indicator of the plant's water status is its water potential, which is a reflection of the balance between water content (pressure potential) and solute concentration (osmotic potential). The plant has the ability to generate a negative osmotic potential in relation to the soil, thereby forcing the absorption of water and, by association, the absorption of nutrients. These ions accumulate in the vacuoles of the plant, which in turn favors the entry of water into these vacuoles, thus maintaining a water reservoir and preserving the plant's turgor. Proton pumps are essential in this process since both nutrient uptake and accumulation in the vacuole occur through specific transporters, which require the generation of an electrochemical gradient of protons, a process that falls on the proton pumps (H + -ATPase). Thus, there are recent studies on the plasma membrane (PM) H+-ATPase pump, a prominent proton pump found in the plant cell membrane. This pump plays a crucial role in generating an electrochemical gradient by pumping protons outwards, an essential process in the generation of energy required for nutrient and water uptake culminating in cell growth. It has been shown that the activation of the PM H+-ATPase involves the modification of its auto-inhibitory terminal domains, suggesting a possible regulation of proton pumping due to physiological factors that interact with these regulatory terminals. On the other hand, it has been shown that various signals that affect plant growth also interfere with the post-translational regulation of this enzyme (Zhang et al., 2021).

In another recent study, overexpression of a single rice gene, known as Oryza sativa plasma membrane (PM) H+-ATPase 1 (OSA1), was shown to improve ammonium uptake and assimilation in roots, while increasing stomatal aperture. This resulted in a 33% increase in grain yield and a 46% improvement in overall nitrogen use efficiency. Since PM H+-ATPase is highly conserved in plants, this enzyme appears as a promising microbially inducible target for more effective nitrogen uptake and carbon sequestration, constituting an essential tool to promote food security and sustainability in agriculture (Falhof et al., 2016).

Once these nutrients have been absorbed, they are stored in the vacuoles or transported to the aerial part of the plant by the flow of transpiration Subsequently, these nutrients are incorporated into organic compounds (assimilation), which are essential for plant growth. It is therefore important to note that mineral nutrition of the plant requires the loss of water through the process of transpiration.

Thus, the plant must maintain a balance between opening and closing the stomata in order to facilitate the exchange of carbon dioxide and water, giving priority to stomatal closure when water becomes scarce. In addition to regulating stomata opening, as mentioned above, the plant has other mechanisms to conserve water by increasing the internal concentration of solutes, including osmolytes and other organic molecules. It also has antioxidant systems to maintain an overall healthy state and ensure efficient photosynthesis, keeping free radical levels within physiological limits. Having demonstrated the involvement of proton pumps in improving plant nutrition using genetic modification techniques, the question arises whether a similar effect can be achieved without genetic modification. In this regard, there is a growing body of scientific evidence showing that some plant growth promoting bacteria (PGPB) have the ability to influence several aspects of plant physiology simultaneously, including the activity and efficiency of pumps responsible for nutrient uptake. In other words, they employ multiple mechanisms of action that vary according to the needs of the plant, always with the aim to improve plant adaptation (Ilagunamaran and Smith, 2017). The mechanisms of action of PGPB bacteria can be summarised in two groups: direct, when the bacteria or their metabolic products affect the metabolism of the plant (including altering the hormonal balance and stimulating adaptive mechanisms); and indirect, when they synthesise metabolites that facilitate the uptake or mobilisation of nutrients or prevent the growth of pathogenic microorganisms in the plant without altering its metabolism. It should be noted that this list is not exhaustive. In the context of this patent, both types of mechanisms are of great interest. The plant has an adaptable and highly inducible metabolism related to its capacity to face adverse situations throughout its life, and this metabolism can be modified by PGPBs or their metabolic products where we can find elicitor molecules, capable of activating the plant metabolism by interacting with plant receptors, or enzymes capable of mobilising soil nutrients, or inhibiting the growth of pathogens.

Within the PGPB bacteria are numerous representatives of the genus *Bacillus,* which currently comprises a total of 239 species and subspecies, ranging from pathogens to extremely beneficial strains in agriculture (Patel and Gupta, 2020). Following the 2009 Bergeys Manual taxonomic classification, the genus Bacillus is placed in the Domain Bacteria, Phylum Firmicutes, Class Bacilli, Order Bacillales and Family Bacillaceae (Logan NA, De Vos P. Genus Bacillus Cohn 1872. In: De Vos P, Garrity M, Jones D, Krieg R and Ludwig W (eds). Bergey's Manual of Systematic Bacteriology. New York: Springer; 2009. pp.21-128). The genus is organised into operational groups, one of which is *Bacillus amyloliquefaciens.*

Recent advances in genomic analysis have increased interest in the biotechnological potential of bacterial species of the *Bacillus amyloliquefaciens* operative group, some of whose members are recognised as plant growth promoting bacteria (PGPB) due to the ability of certain isolates to perform nitrogen fixation, solubilise phosphate, produce siderophores and phytohormones, as well as synthesise antimicrobial compounds. In addition, they are known for their production of various enzymes such as α-amylase, protease, lipase, cellulase, xylanase, pectinase, aminotransferase, barnase, peroxidases and laccase. These bacteria also produce antimicrobial compounds, including non-ribosomal peptides and polyketides, capable of inhibiting the growth of pathogens. Genomic analysis has also revealed potential applications of members of the *Bacillus amyloliquefaciens* task force in the elimination of organophosphorus pesticides (OPs) (Ngalimat et al., 2021). In relation to the *Bacillus amyloliquefaciens* task force, innovative technologies have made it possible to describe new impacts on plant nutrition and carbon dioxide sequestration using an isolated strain of *Bacillus nakamurai,* or the metabolic compounds produced in the growth of the strain; this bacterium belongs to the *Bacillus amyloliquefaciens* task force.

As *Bacillus nakamurai* is a very recent species, there is currently no description of its PGPB potential under water stress conditions, nor are there any published patents on strains of this species for application to plants under such conditions.

### INVENTION. -

The invention for which, in view of the aforementioned state of the art, the patent right are sought, concerns two types of interrelated products of a PGPB of the genus *Bacillus: a* specific strain of *Bacillus nakamurai,* which is a newly recently discovered species, and the metabolites generated in its fermentation process, both with capacities to promote mineral nutrition and plant growth.

As a first inventive component, the isolation and characterization of the bacterial strain *Bacillus nakamurai* with deposit number CECT 30581, which is a microorganism of the Gram + bacteria group, is claimed for its proven ability to enhance the mineral nutrition of plants, by stimulating the absorption of nitrogen, phosphorus, potassium and manganese from the soil, as well as the photosynthetic process, by stimulating the fixation of CO₂ from the air and transpiration, optimizing the capture of energy and the efficiency in the use of water, which translates into an improvement in the growth and production of crops, even under conditions of water stress.

And as a second component of the invention, the separation/extraction of the Total Metabolic Fluid (TMF) of the strain and the identification of the metabolites responsible for its effects; metabolites that will serve as an unequivocal identifying profile of the TMF for its different uses.

Once isolated, the physiological characteristics and genetic analysis of *B. nakamurai* CECT 30581 allowed it to be unequivocally identified, differentiated from other species of the Bacillus genus, and from the other subspecies of the *Bacillus amyloliquefacien* operative group.

To study and characterise the metabolites generated during bacterial growth, which form a complex mixture collectively known as Total Metabolic Fluid (TMF), a multi-stage fractionation process was carried out based on the different polarity of the compounds. First, the TMF was extracted by partitioning, obtaining a fraction known as the HF, which represents the "Hexanic Fraction", in which the hexane-soluble compounds are separated from the aqueous culture medium. However, due to the complexity and diversity of compounds present in TMF and HF, it was necessary to continue the separation process to elucidate the exact composition of the metabolites, performing a more detailed and specific analysis on an even more refined fraction, called HS, which corresponds to the "Hexanic Subfraction". This HS subfraction was obtained by subjecting the HF to a new fractionation process based on the polarity and size of the molecules.

Due to its low polarity nature, the composition of the HS extract was examined using the technique of gas chromatography coupled with mass spectrometry, and to ensure that all compounds were detected, an analysis was carried out using the extract derivatized with BSTFA (N,O-trimethylsilyl bis-trifluoroacetamide). This compound was used to transform the polar hydroxyls into silicon ethers, which are less polar and volatile, which facilitates analysis by mass chromatography. The result has been the identification of the following metabolites generated during the growth of *B. nakamurai* CECT 30581, largely indicative of an intense terpene metabolism:
- Glycerol;
- Prenol;
- Nonanoic acid;
- Diethylene glycol;
- Ethoxybenzoate;
- Dodecane;
- Dodecanoic acid;
- Triethylglycol;
- Myristic acid;
- Mannopyrasone;
- Talopyrasone;
- Palmitic acid;
- Stearic acid;
- Squalene;
- Hexadecane and other hydrocarbons.

It is important to note that the compositional analysis is mainly focused on HS, as it represents a more manageable and specific sample of the metabolites of interest. Once the indicated compounds were identified and characterized in HS, their presence was confirmed in both TMF and HF. This rigorous analytical approach is essential to unveil the chemical complexity of the metabolites in the culture broth and understand their potential biological impact.

The biological effects of the four resulting bacterial products: *B. nakamurai,* TMF, HF and HS, which imply the aforementioned improvement in mineral nutrition and photosynthetic efficiency of plants, have been tested on different plant species, such as tomato, rice or strawberry plants, by measuring the levels of nitrogen, phosphorus, potassium and manganese of the treated plants, as well as the parameters related to photosynthetic efficiency, net photosynthesis rate, photosystem ii efficiency (èPSR), water use efficiency (WUE), dry weight, fresh weight and plant height. They have also been tested under water stress conditions, created by a reduction in irrigation water, in plants such as olive or blueberry, an increase in production (kg/ha) is found.

Initially, experiments were carried out on tomato plants where the three types of substances: TMF HF and HS were applied at the root level (Trials 1 and 2 of the Method of Implementation section), observing a significant increase in the photosynthesis rate, which is an important indicator of the efficiency of the plant to convert sunlight into chemical energy, which in turn can have a positive effect on biomass production and crop quality. In fact, significant increases were recorded in several growth-related parameters, such as height, fresh weight and dry weight compared to the control group. A notable increase in the concentration of nitrogen, phosphorus, potassium and manganese in the aerial part of the plants was also detected. These findings suggested that the application of the new metabolic products has a positive effect on the growth and nutrition of the treated plants.

Similar trials were then carried out on rice, applying TMF and HF and HS at the root level (Trials 3 and 4). A significant increase in the net photosynthesis rate was also observed. In addition, increases were recorded in photosystem II efficiency (ePSR), a key indicator of photosynthesis, and in water use efficiency (WUE), which means that plants use solar energy and water more effectively for their growth and development. In terms of growth, the plants experienced a significant increase in height and fresh weight, and also in nitrogen, phosphorus, potassium and manganese, all of which confirms that the application of the three metabolic products has a positive effect on plant growth and nutrition.

In another experiment with rice plants, a study of the efficient concentration of the hexanic fraction and subfraction (Trial 5) was carried out. HF and HS were applied at the root level, using three different concentrations of these extracts. First, it was observed that certain specific concentrations of both had a significant impact on the net photosynthesis rate, indicating an improvement in the plants' ability to convert sunlight into useful energy, therefore, in their ability to grow and develop better. In addition, a substantial increase in the levels of nitrogen, phosphorus, potassium and manganese in the aerial part was also recorded, suggesting that the application of both extracts on the roots promotes the absorption and mobilization of these nutrients from the soil, essential for their healthy development, and their availability at high levels can contribute to better crop yield and quality.

In order to validate and reinforce the results obtained in the previous trial, a second experiment was carried out in which the hexanic fraction and subfraction were again applied at the root level in the same rice plants, using three other different concentrations (Trial 6). This second trial provided data that support and extend the conclusions previously obtained, as significant improvements were detected in transpiration and net photosynthesis, and significant increases in plant height and fresh weight. These results indicate a positive impact on the overall growth and development of the plants, which could be of great relevance in agricultural and crop applications. A notable increase in the levels of nitrogen, phosphorus, potassium and manganese in the aerial part of the plant was also observed.

In another experiment carried out on tomato, B. nakamurai CECT 30581 was applied at the root level (Trial 7), showing a significant increase in the height of the treated plants and in the levels of the indicated nutrients.

### FIGURES AND GRAPHICS. -

At the end of this descriptive report, figures are included with process diagrams and graphs of the experimental results obtained.

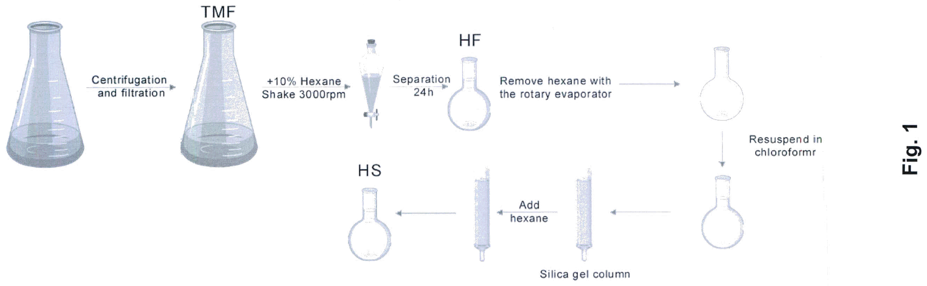

**Figure 1** shows the schematic diagram of the production process of the hexanic fraction (HF) and hexanic subfraction (HS) from the culture broth of the strain.

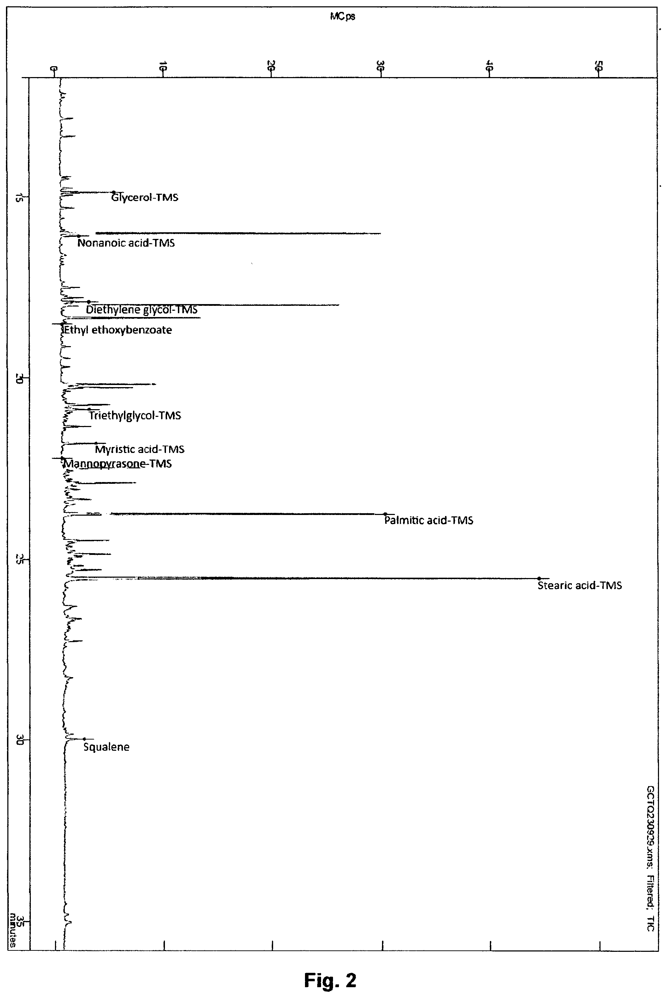

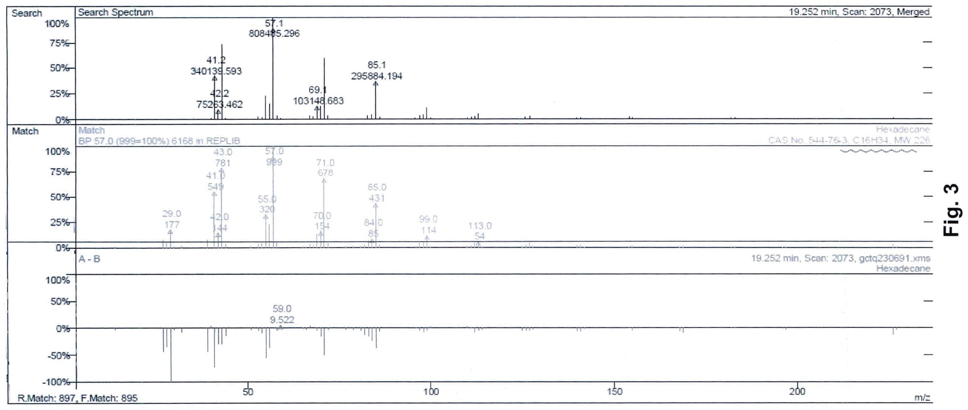

The graph in **Figure 2** is the gas chromatogram of the hexanic subfraction HS, and the graph in **Figure 3** is the mass spectrophotometry of that same sample.

### METHOD OF REALISATION.-

In a bacterial screening carried out in the rhizosphere of *Pinus pinaster,* a strain belonging to the *Bacillus* genus was isolated, which after being identified by a genetic analysis of the 16S gene using the tools available in Ezbiocloud, resulted, with 100% identity, in B. nakamurai. Table 1 shows the result of this analysis.

**TABLE 1**

| Name | *Bacillus nakamurai* |
|---|---|
| Strain | NRRL B-41091 |
| Authors | Dunlap et al. 2016 |
| Taxonomy | Bacteria; Firmicutes; Bacilli; Bacillales; Bacillaceae; Bacillus |
| Accession | LSAZ01000028 |
| Pairwise Similarity (%) | 100 |
| Mismatch/Total nt | 0/1027 |
| Completeness (%) | 100 |

### I. Production of TMF, HF and HS.-

*B. nakamurai* CECT 30581 can be grown in shake-fermentation flasks on a small or large scale (in laboratory or industrial fermenters) using a suitable medium and under conditions that allow cell growth. The culture must contain a suitable nutrient medium comprising carbon and nitrogen sources and inorganic salts, using procedures known in the art. Suitable media are available from commercial sources or can be prepared according to published compositions. After fermentation, a supernatant, filtrate and/or extract of, or derivative of said *B. nakamurai* CECT 30581 can be used as active material to be applied directly to plants, or in compositions to formulate an organic biofertilizer.

The concept of TMF, or "Total Metabolic Fluid ", encompasses all the metabolites generated by the bacteria during its growth process in the indicated fermenter and suitable culture medium. To produce this TMF, the liquid that is produced by the fermenter after the bacteria have completed their growth cycle is used.

The process of obtaining TMF involves a series of steps. First, the bacteria are separated from the liquid using a highly efficient centrifugation process. This centrifugation allows more than 99% of the bacteria present in the liquid to be eliminated. A second stage of the process is carried out using a 0.2 micrometer filter. This filter has the ability to retain even the smallest bacteria, thus ensuring that any remaining bacteria are completely removed from the liquid. Once this second step is completed, a pure liquid is obtained that contains only the molecules and compounds generated by the bacteria during its growth phase, and this liquid is called TMF (Total Metabolic Fluid ).

The production of the Hexanic Fraction (HF) is an essential process that is carried out from the Total Metabolic Fluid (TMF). This process is based on separating the molecules present in the TMF according to their affinity for the solvent hexane compared to their affinity for water. The procedure begins with the addition of 10% of the total volume of the TMF in hexane, an organic solvent with a low affinity for water. The resulting mixture is then subjected to stirring at 3000 rpm for a period of 5 minutes. This stirring allows the molecules present in the TMF to be distributed between hexane and water selectively, based on their polarity. After the stirring is completed, the mixture is left to rest for a period of 24 hours. During this time, the molecules that are more soluble in hexane will dissolve in this phase, while those that have a higher affinity for water will remain in the aqueous phase. After this resting time, the two phases are separated, thus obtaining the Hexanic Fraction (HF). The HF, in this context, represents a highly enriched fraction of molecules that have shown a higher affinity for hexane compared to water. This polarity-based separation is crucial to selectively isolate and concentrate the compounds of interest present in the TMF, facilitating their subsequent study and characterization.

The production of the Hexane Subfraction (HS) is a process that starts from the Hexane Fraction (HF). This procedure is carried out using a molecular separation technique based on the polarity and size of the molecules, using a column packed with silica gel. First, the hexane is removed from the HF through an evaporation process. Then, 0.1% of its volume of chloroform is added in order to resuspend the molecules present in the solution. This mixture is then carefully applied to the silica gel column. A crucial stage of the process involves the subsequent elution of the column. At this point, the same volume of hexane that had been previously removed from the HF is introduced. This has a specific purpose: the hexane acts as an entraining agent, carrying with it the molecules that had been suspended in the chloroform during the previous step. These molecules pass through the silica gel column together with the hexane. Finally, the liquid obtained at the end of the silica gel column contains the molecules that are soluble in hexane and that have been able to pass through the column. This resulting liquid corresponds to the Hexane Subfraction (HS), which is now ready for use or further processing.

**Figure 1** schematically shows this production process of TMF, HF and HS for the isolated bacterial strain.

### II. Analysis HS.-

The chromatographic profile of the sample, reflected in the gas chromatogram in **Figure 2****,** reveals the presence of long-chain compounds (fatty acids, alcohols), sugars, phenolic compounds and mostly hydrocarbons. In the chromatogram, approximately 35 peaks can be identified.

A detailed analysis of the predominant peaks was carried out by mass spectroscopy. The identification of 14 compounds was achieved by comparing the data with the NIST library database and using the Kovats index. These results are summarized in **Table 2.**

The analytical conditions were carried out on a PREM EI&CI equipment, 436GC chromatograph, Rxi-5Sil MS 30×0.25 mm column, 0.25um, flow: 1.2 ml/min in a temperature range of 40-300oC in 60 minutes, electronic ionization: El 70eV from 40 to 650 amu.

**TABLE 2**

| Compound name | Holding time (min) |
|---|---|
| Glycerol | 14,87 |
| Prenol | 15,40 |
| Nonanoic acid | 16,09 |
| Diethylene glycol | 17,89 |
| Ethoxybenzoate | 18,51 |
| Dodecanol | 18,97 |
| Dodecanoic acid | 19,94 |
| Triethylglycol | 20,87 |
| Myristic acid | 21,80 |
| Manopyrasone | 22,22 |
| Thalopyrasone | 23,10 |
| Palmitic acid | 23,74 |
| Stearic acid | 25,54 |
| Squalene | 29,98 |

The hydrocarbons were identified through their fragmentation pattern, which generated an ion at 57 m/z with successive losses of CH₃ methyl group (15 u). The graph in **Figure 3** presents the mass spectrum obtained for hexadecane.

### Ill. Demonstrative trials of the capacity of B. nakamurai, TMF, FH, and SH as mineral nutrition enhancers (nitrogen, phosphorus, potassium and manganese), and stimulants of the photosynthetic process, promoting an increase in growth in terms of dry weight and improving the efficiency in the use of water. -

**Trial 1:** *Solanum lycopersicum* var. San Pedro, TMF and FH - Direct inoculation at root level of TMF and FH (Control, TMF, FH) in tomato (*Solanum lycopersicum* var. San Pedro). Experiment carried out in an experimental chamber under controlled conditions of light, humidity and temperature on a total of 84 plants and N = 3, with a randomised block model. Tomato plants were inoculated with TMF and FH at 4 weeks after germination. At 6 weeks, photosynthetic parameters were measured, recording a significant increase in net photosynthesis in plants treated with TMF with respect to control plants; a biometry analysis recorded a significant increase in plants treated with TMF and FH with respect to control plants in height, dry weight and fresh weight; Finally, a nutritional analysis was carried out, recording a significant increase in nitrogen, phosphorus, potassium and manganese levels in plants treated with TMF and FH compared to control plants.

**Trial 2:** *Solanum lycopersicum* var. San Pedro, SH.- Direct inoculation at the root level of SH (Control & SH) in tomato (Solanum lycopersicum var. San Pedro). Experiment carried out in an experimental chamber under controlled conditions of light, humidity and temperature on a total of 24 plants and an N = 3, with a random block model. 2 inoculations of the hexanic subfraction were carried out in tomato plants, at 2 and 3 weeks after germination. After 4 weeks the photosynthetic parameters were measured, recording a significant increase in the plants treated with SH with respect to the control plants in the efficiency of photosystem II (èPSR); a biometry analysis recording a significant increase in the plants treated with HS with respect to the control plants in dry weight; Finally, a nutritional analysis was performed, recording a significant increase in the levels of nitrogen, phosphorus, potassium, and manganese in the plants treated with HS compared to the control plants.

**Trial 3:** *Oryza sativa* var. Bomba, TMF and FH - Direct inoculation at root level of TMF and FH in rice (*Oryza sativa* var. Bomba). Experiment carried out in an experimental chamber under controlled conditions of light, humidity and temperature on a total of 450 plants and N = 3, with a randomised block model. TMF and fraction were inoculated on rice plants at the first and second week of germination. At 3 weeks, photosynthetic parameters were measured and a significant increase in net photosynthesis, photosystem II efficiency (éPSR) and water use efficiency (WUE) was recorded in TMF-treated plants compared to control plants; A biometric analysis showed a significant increase in height and fresh weight of TML-treated plants compared to control plants. Finally, a nutritional analysis showed a significant increase in nitrogen, phosphorus, potassium and manganese levels in TMF-treated plants compared to control plants.

**Trial 4:** *Oryza sativa* var. Bomba, SH.- Direct inoculation at the root level of HS in rice (*Oryza sativa* var. Bomba). Experiment carried out in an experimental chamber under controlled conditions of light, humidity and temperature on a total of 300 plants and an N = 3, with a random block model. Two inoculations of the hexanic subfraction were carried out in rice plants, at 2 and 3 weeks after germination. At 4 weeks the photosynthetic parameters were measured without observing significant changes in the treated plants with respect to the control plants; a biometry analysis recorded a significant increase in the plants treated with SH with respect to the control plants in fresh weight; finally, a nutritional analysis was carried out, recording a significant increase in the plants treated with HS with respect to the control plants in the levels of nitrogen, phosphorus, potassium, and manganese.

**Trial 5:** *Oryza sativa* var. Bomba, HF and HS, efficient concentration study.- Direct inoculation at the root level in rice (*Oryza sativa* var. Bomba) at the root level of HF and HS with three different concentrations (Control, HF ([1:1], [1:10], [1:100]) and HS ([1:1], [1:10], [1:100]) hexanic, v:v). Experiment carried out in an experimental chamber under controlled conditions of light, humidity and temperature on a total of 105 plants and an N = 3, with a random block model. 2 inoculations of SH were carried out in rice plants, at 2 and 3 weeks after germination. After 5 weeks the photosynthetic parameters were evaluated, observing significant changes in the plants treated with HF [1:10] and SH [1:10] with respect to the control plants in net photosynthesis; a biometric analysis without observing significant changes in the treated plants with respect to the control plants; finally, a nutritional analysis was carried out, recording a significant increase in the plants treated with HF [1:10] and HS [1:10] with respect to the control plants in the levels of nitrogen, phosphorus, potassium, and manganese

**Trial 6:** *Oryza sativa* var. Ricetech, FH and SH optimum concentration - Direct inoculation at root level in rice (*Oryza sativa* var. Ricetech) at root level of FH and SH with three different concentrations (Control, fraction ([1:1], [1:10], [1:100]) and subfraction ([1:1], [1:10], [1:100]) hexanic). Experiment carried out in an experimental chamber under controlled conditions of light, humidity and temperature on a total of 105 plants and an N = 3, with a randomised block model. Two inoculations of the SH were carried out on rice plants, 2 and 3 weeks after germination. At 5 weeks, photosynthetic parameters were evaluated and significant changes of FH [1:1] and SH [1:1] treated plants with respect to control plants were observed in transpiration and net photosynthesis; a biometry analysis revealed significant changes of FH [1:1] and SH [1:1] with respect to control plants in height and fresh weight; finally, a nutritional analysis showed a significant increase of plants treated with hexane FH [1:1] and SH [1:1] with respect to control plants in the levels of nitrogen, phosphorus, potassium and manganese.

**Trial 7:** *Solanum lycopersicum* var. San Pedro, *B. nakamurai* CECT 30581.- Direct inoculation at the root level of *B. nakamurai* CECT 20581 in tomato (*Solanum lycopersicum* var. San Pedro). Experiment carried out in an experimental chamber under controlled conditions of light, humidity and temperature on a total of 42 plants and an N = 3 with a random block model. Two inoculations of *B. nakamurai* were carried out at 2 and 4 weeks after the appearance of the first true leaf. After 8 weeks the biometric parameters (Height) were evaluated, observing significant increases in both parameters of the treated plants with respect to the control plants; finally, a nutritional analysis was carried out, recording a significant increase in the treated plants with respect to the control plants in the levels of nitrogen, phosphorus, potassium, and manganese.

**Trial 8:** *Olea europaea* var. Arbequina, *B. nakamurai* CECT 30581, with and without water restriction.- Direct inoculation at root level of *B*. *nakamurai* CECT 20581 in olive trees (*Olea europaea* var. Arbequina). Applications were made every 15 days from April to September (12 applications). The number of plants in the trial was 20 per treatment plus the control, with both irrigation regimes (100% water and 75% water). In the part of the trial referring to normal conditions, it was observed that *B*. *nakamurai* caused an increase in production (kg/ha) of 4% over the control, and an increase in fat yield (0.4%) was also recorded. In the part referring to water limitation during the summer months (25%), it was observed that *B*. *nakamurai* caused an increase in production (kg/ha) of 20.7% over the control, an increase in fat yield was also recorded (17.7%).

**Trial 9:** *Vaccinium corymbosum* var. Cupla *B. nakamurai* CECT 30581.- Direct inoculation at root level of *B*. *nakamurai* CECT 20581 in blueberry (*Vaccinium corymbosum* var. Cupla). Production improvement experiment with open-air irrigation limitation in blueberry. Experiment carried out under real field production conditions on a total of 21 plants per treatment (n=3, 7 plants per replicate) for each treatment (control and bacteria), with a random block experimental model. Cell suspensions of the strain were applied at root level during the 10-month production cycle, twice a month, from October to September; water was limited by 25% throughout the cycle. Total production was collected. An increase in fruit production (+11%) was recorded in the treated plants compared to the control.

**Trial 10:** *Fragaria vesca* var. Fortuna, *B. nakamurai* CECT 30581 and LMT.- Direct inoculation at root level of *B. nakamurai* CECT 20581 and TMF on *Fragaria vesca* var. Fortuna. Experiment focused on intensive strawberry production, with the aim of boosting production. In this study, the effects of the bacteria *B. nakamurai* CECT 20581 and its TML have been evaluated through monthly applications at root level, carried out from October to May. The experiment was carried out in production greenhouses, using 60 plants per treatment. The results obtained reveal a notable increase in strawberry production, observing an increase of 138% in plants treated with TMF, while those treated with the *B. nakamurai* strain experienced an increase of 72%.

### References cited:

Falhof, J., Pedersen, J. T., Fuglsang, A. T., & Palmgren, M. (2016). Plasma membrane H+-ATPase regulation in the center of plant physiology. Molecular plant, 9(3), 323-337.
Ilangumaran, G., & Smith, D. L. (2017). Plant growth promoting rhizobacteria in amelioration of salinity stress: a systems biology perspective. Frontiers in Plant Science, 8, 1768.
Ngalimat, M. S., Yahaya, R. S. R., Baharudin, M. M. A. A., Yaminudin, S. M., Karim, M., Ahmad, S. A., & Sabri, S. (2021). A review on the biotechnological applications of the operational group Bacillus amyloliquefaciens. Microorganisms, 9(3), 614.
Patel, S., & Gupta, R. S. (2020). A phylogenomic and comparative genomic framework for resolving the polyphyly of the genus Bacillus: Proposal for six new genera of Bacillus species, Peribacillus gen. nov., Cytobacillus gen. nov., Mesobacillus gen. nov., Neobacillus gen. nov., Metabacillus gen. nov. and Alkalihalobacillus gen. nov. International journal of systematic and evolutionary microbiology, 70(1), 406-438.
Zhang, M., Wang, Y., Chen, X., Xu, F., Ding, M., Ye, W., ... & Zhu, Y. (2021). Plasma membrane H+-ATPase overexpression increases rice yield via simultaneous enhancement of nutrient uptake and photosynthesis. Nature Communications, 12(1), 735.

## Claims

1. **Bacterial strain of the species *Bacillus nakamurai* with deposit number CECT 30581,** a microorganism of the group of Gram + bacteria, genus *Bacillus,* **characterized by** its ability to stimulate the absorption of nitrogen, phosphorus, potassium and manganese in plants under conditions of water stress, and to optimize energy capture and water use efficiency under these conditions.

2. **Total metabolic fluid (TMF),** derived from the fermentation in the culture medium of the bacterial strain *Bacillus nakamurai* with deposit number CECT 30581, with the following component metabolites: glycerol, prenol, nonanoic acid, diethylene glycol, ethoxybenzoate, dodecane, dodecanoic acid, triethyl glycol, myristic acid, mannopyrasone, talopyrasone, palmitic acid, stearic acid, squalene, hexadecane and other hydrocarbons, obtained by a process of separating the bacteria from the fermentation supernatant through two operations: (1) centrifugation and (2) filtration with a 0.2 µm filter, **characterized by** its capacity to stimulate the absorption of nitrogen, phosphorus, potassium and manganese in plants under conditions of water stress, and to optimize energy uptake and water use efficiency under these conditions.

3. **Hexanic fraction (HF)** of the total metabolic fluid (TMF) from the fermentation in the culture medium of the bacterial strain *Bacillus nakamurai* with deposit number CECT 30581, with the following component metabolites: glycerol, prenol, nonanoic acid, diethylene glycol, ethoxybenzoate, dodecane, dodecanoic acid, triethyl glycol, myristic acid, mannopyrasone, talopyrasone, palmitic acid, stearic acid, squalene, hexadecane and other hydrocarbons, obtained by a process comprising four operations: (1) addition of 10% of the total volume of the MTF in hexane until the mixture is complete, (2) stirring at 3000 rpm for 5 minutes, (3) resting for 24 hours and (4) separation of the HF from the aqueous phase, **characterized by** its capacity to stimulate the absorption of nitrogen, phosphorus, potassium and manganese in plants under water stress conditions, and by optimize energy capture and water use efficiency under these conditions.

4. **Hexanic subfraction (HS)** of the total metabolic fluid (TMF) from the fermentation in the culture medium of the bacterial strain *Bacillus nakamurai* with deposit number CECT 30581, with the following component metabolites: glycerol, prenol, nonanoic acid, diethylene glycol, ethoxybenzoate, dodecane, dodecanoic acid, triethylglycol, myristic acid, mannopyrasone, talopyrasone, palmitic acid, stearic acid, squalene, hexadecane and other hydrocarbons, obtained from the hexane fraction (HF) of the TMF by a process comprising five operations: (1) elimination of hexane from the HF in a rotary evaporator, (2) resuspension in chloroform, adding 0.1% of its volume of chloroform, (3) application of the resulting mixture on a silica gel column, (4) elution of the column with addition of hexane, in the same volume removed from the HF, and (5) extraction of the liquid corresponding to the SH contained at the end of the column, **characterized by** its capacity to stimulate the absorption of nitrogen, phosphorus, potassium and manganese in plants under conditions of water stress, and by optimize energy capture and efficiency in the use of water under these conditions.

5. Use of the bacterial strain of the species *Bacillus nakamurai* with deposit number CECT 30581, or of the total metabolic fluid (TMF), the hexanic fraction (HF) or hexanic subfraction (HS) of the culture medium of the strain, according to claims 1 to 4, for its application in any species of plants, such as olive or blueberry plants, to improve plant growth and production under conditions of water stress, due to drought or due to reduction of irrigation water.

6. Use of the bacterial strain of the species *Bacillus nakamurai* with deposit number CECT 30581, or of the total metabolic fluid (TMF), the hexanic fraction (HF) or hexanic subfraction (HS) of the culture medium of the strain, according to claim 5, forming part of any bacterial preparation, either individually or in combination with other organisms, and by any available means that puts the bacteria or its metabolites in contact with the seed, the radical or aerial system of the plants.
